# EUROPEAN PATENT SPECIFICATION

(11) **EP 4 154 843 B1**
(45) Date of publication and mention of the grant of the patent: **24.12.2025**
(21) Application number: 21808359.0
(22) Date of filing: 18.03.2021
(51) Int. Cl.: A61C 8/00, A61B 34/10, A61B 34/00, A61B 90/00

(54) **MAXILLARY SINUS AUGMENTATION SIMULATION METHOD AND APPARATUS THEREFOR**
SIMULATIONSVERFAHREN UND -VORRICHTUNG FÜR DIE OBERKIEFERSINUSVERGRÖSSERUNG
PROCÉDÉ DE SIMULATION DE SURÉLÉVATION DU SINUS MAXILLAIRE ET DISPOSITIF S'Y RAPPORTANT

(30) Priority: 19.05.2020 KR 20200059741
(43) Date of publication of application: 29.03.2023
(73) Proprietor: Osstem Implant Co., Ltd., Gangseo-gu Seoul 07789 (KR)
(72) Inventor: JOO, Won Hyeong, Seoul 08323 (KR); LEE, Do Hyun, Seoul 06968 (KR); CHOI, Kyoo Ok, Seoul 07789 (KR)
(74) Representative: Klunker IP Patentanwälte PartG mbB
(86) International application number: PCT/KR2021/003319
(87) International publication number: WO 2021/235664

(56) References cited:
- WO-A1-2014/150257
- KR-A- 20190 077 859
- KR-B1- 101 212 556
- KR-B1- 101 626 347
- KR-B1- 101 762 655
- KR-B1- 101 762 655
- GHOSN NABIL ET AL: "Computer-Assisted Analysis of Bone Volume for Sinus Augmentation Procedure", INTERNATIONAL ARAB JOURNAL OF DENTISTRY : IAJD, SAINT-JOSEPH UNIVERSITY OF BEIRUT, LEBANON, vol. 7, no. 3, 1 January 2016 (2016-01-01), pages 95 - 108, XP009531994, ISSN: 2709-4774
- GHOSN, NABIL; KHOURY, JOE; NAAMAN, NADA: "Computer-Assisted Analysis of Bone Volume for Sinus Augmentation Procedure", INTERNATIONAL ARAB JOURNAL OF DENTISTRY, vol. 7, no. 3, 1 January 2016 (2016-01-01), Lebanon, pages 95 - 108, XP009531994, ISSN: 2709-4774

## Description

### [Technical Field]

The present disclosure relates to a dental image processing technology, and more particularly, to a technology of simulating maxillary sinus lifting in a dental image.

### [Background Art]

An implant is a dental prosthesis used to fix an artificial tooth by placing a implant into or on the jawbone to support a crown for filling a damaged tooth. For successful implant surgery, it is important to firmly fix an implant to a bone. However, when the bone tissue is weak or the bone thickness is insufficient at a position where the implant will be placed, implant surgery is performed after reinforcing the bone tissue by the grafting of an autogenous bone, an artificial bone, a synthetic bone, or the like.

In particular, when performing implant surgery at a position below the maxillary sinus, in many cases, the bone thickness is insufficient relative to an implant length, which makes it difficult to place the implant. Here, when implant surgery is performed despite the insufficient bone thickness, since the surrounding bone tissue cannot sufficiently support the placed implant, a problem that the implant falls out, or the surrounding bone tissue breaks down during masticatory movement, may occur. In order to prevent such a problem, when the thickness of maxillary bone is insufficient, maxillary sinus lifting in which the maxillary sinus membrane is elevated and then a bone is grafted into the maxillary sinus to secure an implant placement space is performed. The maxillary sinus membrane tissue is a tissue formed in the maxillary sinus to control humidity in the nasal cavity and control resonance during vocalization.

KR 101 762 655 B1 discloses an implant surgery planning method and device using simulation to plan implant surgery involving sinus lift, which includes extracting sinus regions from volume images, removing overlapping areas with the fixture placement region to acquire new image data, and calculating the bone graft amount based on the volume of the remaining region.

GHOSN NABIL ET AL: "Computer-Assisted Analysis of Bone Volume for Sinus Augmentation Procedure" International Arab Journal of Dentistry: IAJD, SAINT-JOSEPH UNIVERSITY OF BEIRUT; LEBANON; vol. 7, no. 3, 1 January 2016 (2016-01-01), pages 95-108, XP009531994, ISSN: 2709-4774, discloses a computer-assisted analysis method for determining the bone volume required for sinus augmentation, involving CBCT scans and the Simplant Pro software to perform preoperative simulations, calculate graft volumes, and assess the accuracy and reliability of these calculations against the actual volume used during surgery.

However, in general dental image processing software, during mplant placement simulation, a user should directly check whether maxillary sinus involvement by an implant occurs. In this case, it is inconvenient to use, and during maxillary sinus lifting surgery, there is a risk of errors due to a user performing the surgery based on the user's own experience and judgement.

### [Disclosure]

### [Technical Problem]

One embodiment of the present disclosure proposes a maxillary sinus lifting simulation method and an apparatus therefor capable of, during i placement simulation, automatically performing maxillary sinus lifting simulation to inform a user of whether maxillary sinus involvement by an implant occurs, thereby improving convenience of use and reducing errors.

### [Technical Solution]

The invention is set out in the appended set of claims.

### [Advantageous Effects]

According to a maxillary sinus lifting simulation method and apparatus therefor according to one embodiment, during implant placement simulation, maxillary sinus lifting simulation is automatically performed to provide information on an area where maxillary sinus lifting is necessary and information on an amount of bone graft to a user. **In** this way, the user can establish a plan for maxillary sinus lifting and bone grafting surgery by referring to the provided pieces of information.

Since the information on the area where maxillary sinus lifting is necessary and the information on the amount of bone graft are proposed to the user, the user can prepare for surgery after checking a necessary amount of bone graft in advance in the process of establishing the surgical plan and can make good use of the pieces of information even during the actual surgery.

Also, since maxillary sinus lifting simulation information is automatically changed and provided in response to a change in an implant placement situation such as the position or length of an implant, it helps the user reduce errors while easily using the provided maxillary sinus lifting simulation information.

Further, since a screen for setting maxillary sinus lifting simulation is provided and a user interface is provided on the screen, user convenience can be improved.

### [Description of Drawings]

FIG. 1 is a view illustrating a configuration of a maxillary sinus lifting simulation apparatus according to one embodiment of the present disclosure.
FIG. 2 is a view illustrating image screens according to a maxillary sinus lifting simulation process according to one embodiment of the present disclosure.
FIG. 3 is a view illustrating image screens according to a maxillary sinus lifting simulation process according to another embodiment of the present disclosure.
FIG. 4 is a view illustrating a setting screen for maxillary sinus lifting simulation and a use example thereof according to one embodiment of the present disclosure.
FIG. 5 is a view illustrating image screens showing movement of a maxillary sinus simulation area according to movement of an implant according to one embodiment of the present disclosure.
FIG. 6 is a view illustrating image screens displaying movement of an implant involvement area and a change in an amount of bone graft according to movement of an implant.
FIG. 7 is a view illustrating image screens of the implant involvement area according to a change in the length of the implant according to one embodiment of the present disclosure.
FIG. 8 is a view illustrating a screen automatically displaying maxillary sinus lifting simulation information in a 3D image according to one embodiment of the present disclosure.
FIG. 9 is a view illustrating image screens in a case in which screen showing is selected by a user according to one embodiment of the present disclosure.
FIG. 10 is a view illustrating image screens in a case in which screen hiding is selected by the user according to one embodiment of the present disclosure.
FIG. 11 is a view illustrating image screens for maxillary sinus lifting simulation according to one embodiment of the present disclosure.
FIG. 12 is a view illustrating a flowchart of a maxillary sinus lifting simulation method according to one embodiment of the present disclosure.

### [Modes of the Invention]

Advantages and features of the present disclosure and methods of achieving the same should become clear from embodiments described in detail below with reference to the accompanying drawings. However, the present disclosure is not limited to the embodiments disclosed below and may be implemented in various different forms. The embodiments herein only make the disclosure of the present disclosure complete and are provided to completely inform those of ordinary skill in the art to which the present disclosure pertains of the scope of the disclosure. The present disclosure is defined only by the scope of the claims. Like components are denoted by like reference numerals throughout the specification.

In describing the embodiments of the present disclosure, when detailed description of a known function or component is determined as having the possibility of unnecessarily obscuring the gist of the present disclosure, the detailed description will be omitted, and the terms used herein are terms defined in consideration of functions in the embodiments of the present disclosure and may vary according to an intention or practice of a user or an operator. Therefore, the terms should be defined on the basis of the content throughout the specification.

Since combinations of blocks of the accompanying block diagram and operations of the accompanying flowchart may be performed by computer program instructions (execution engines) and the computer program instructions may be embedded in processors of general purpose computers, special purpose computers, or other programmable data processing apparatuses, the instructions that are executed by the processors of the computers or other programmable data processing apparatuses generate means for performing functions described in the blocks of the block diagram or the operations of the flowchart.

Since the computer program instructions may be stored in computer usable or computer readable memories capable of being used in computers or other programmable data processing apparatuses to realize functions in particular manners, the instructions stored in the computer usable or computer readable memories can also produce manufacturing items that include instruction means configured to perform the functions described in the blocks of the block diagram or the operations of the flowchart.

In addition, since the computer program instructions may be embedded in the computers or other programmable data processing apparatuses, the instructions, which perform a series of operations in the computers or the other programmable data processing apparatuses to generate computer-executed processes and execute the computers or the other programmable data processing apparatuses, may also provide operations for executing the functions described in the blocks of the block diagram and the operations of the flowchart.

In addition, the blocks or operations may represent portions of modules, segments, or codes including one or more executable instructions for executing specified logical functions, and in some alternative embodiments, the functions described in the blocks or operations may also be performed out of order. For example, two blocks or operations which are sequentially described may also be simultaneously performed, or the blocks or operations may also be performed in reverse order of the corresponding functions as necessary.

Hereinafter, embodiments of the present disclosure will be described in detail with reference to the accompanying drawings. However, the embodiments of the present disclosure which will be described below may be modified in several different forms, and the scope of the present disclosure is not limited to the embodiments to be described below. The embodiments of the present disclosure are provided to fully explain the present disclosure to those of ordinary skill in the art.

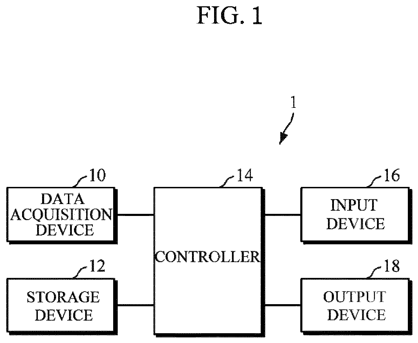

FIG. 1 is a view illustrating a configuration of a maxillary sinus lifting simulation apparatus (hereinafter referred to as a "simulation apparatus") according to one embodiment of the present disclosure.

Referring to FIG. 1, a simulation apparatus 1 according to one embodiment establishes an implant placement plan by designing parts such as a crown, an implant, and an abutment for dental treatment. A user may simulate the established implant placement plan and then perform implant surgery. In particular, among surgical cases, when planning implant placement in a maxillary tooth, implant surgery may be performed by checking a maxillary sinus area in a dental image to prevent perforation of the maxillary sinus, predicting a case in which maxillary sinus lifting is necessary, performing the maxillary sinus lifting when necessary, and then placing the implant.

Segmentation and volume measurement functions are provided for a maxillary sinus area through a dental image program using a 3D volume rendering technique of computed tomography (CT) image data, which is a dental image. In this way, the user may check the maxillary sinus area in 2D/3D images. The simulation apparatus 1 according to one embodiment performs implant placement simulation for placing an implant in a dental image and, in a case in which the implant placed during the implant placement simulation involves the maxillary sinus, automatically performs maxillary sinus lifting simulation and displays maxillary sinus lifting simulation information in the dental image. The maxillary sinus lifting simulation information includes information on an area where maxillary sinus involvement occurs based on the implant and information on an amount of bone graft. The information on the amount of bone graft may be acquired by calculating the volume of an implant involvement area. In this way, by automatically displaying the implant involvement area when the implant involves the maxillary sinus, it is possible to address problems that may occur when the user performs maxillary sinus lifting and implant surgery based on the user's own experience and judgement.

The simulation apparatus 1 is an electronic apparatus that executes image processing software for performing simulation in a dental image. Examples of the electronic apparatus include a computer, a laptop, a tablet personal computer (PC), a smartphone, a mobile phone, a personal media player (PMP), a personal digital assistant (PDA), and the like. Examples of the image processing software include a guide design program, implant simulation software, scan software, computer-aided design (CAD) software, and the like. Also, the image processing software may be applied as software for processing general dental images other than those for use in dental implant surgery.

A dental guide design process using the image processing software consists of registering a patient to receive surgery, acquiring CT data and oral scan data of the registered patient, matching the CT data and the oral scan data, generating a dental arch line in the matched image data, generating a panoramic image using the dental arch line, setting the position and size of a crown model in the oral scan data of the patient, setting the position of an implant structure including an implant in the CT data of the patient, designing the shape of a guide, and outputting the final guide. The present disclosure relates to a technology of automatically providing maxillary sinus lifting simulation information in response to maxillary sinus involvement by an implant in an implant placement simulation process during the above process.

Referring to FIG. 1, the simulation apparatus 1 according to one embodiment includes a data acquisition device 10, a storage device 12, a controller 14, an input device 16, and an output device 18.

The data acquisition device 10 acquires dental image data from teeth including a damaged target tooth of a patient. Examples of the dental image data include CT image data and oral scan data of the patient.

The oral scan data is data containing information on the actual teeth including the damaged tooth and may be 3D information. The oral scan data may be acquired by scanning a plaster model of an oral cavity of the patient with a 3D scanner. As another example, the oral scan data may be acquired by scanning the inside of the oral cavity of the patient using a 3D intra-oral scanner. The acquired oral scan data may be stored in the storage device 12.

The CT image data may be acquired by generating tomographic images of the patient's head using CT, segmenting boundaries of teeth in each tomographic image, and then combining the segmented boundaries into one. The oral scan data and the CT image data include an image obtained by imaging maxillary teeth from below the maxillary teeth with the patient's mouth open, an image obtained by imaging mandibular teeth above the mandibular teeth with the patient's mouth open, an image obtained by imaging a local area with the patient's mouth closed, an oral radiograph, and the like. The obtained CT image data may be stored in the storage device 12.

Various pieces of data such as information necessary to perform operations of the simulation apparatus 1 and information generated according to performing the operations are stored in the storage device 12. Pieces of oral scan data and CT image data of individual patients may be stored in the storage device 12 according to one embodiment, and during dental treatment simulation, oral scan data and CT image data of a specific patient, among all the pieces of oral scan data and CT image data, may be provided to the controller 14 according to a user request. Here, maxillary teeth arrangement images and mandibular teeth arrangement images of individual patients may be stored in the storage device 12, and a maxillary teeth arrangement image and a mandibular teeth arrangement image which match the oral scan data and CT image data of the specific patient may be provided to the controller 14 according to a user request.

The controller 14 controls each functional component while performing implant placement simulation and maxillary sinus lifting simulation through control using a computer program. The controller 14 manages screen information displayed on a screen through the output device 18 and performs the implant placement simulation in which a virtual implant object is placed in a dental image. The dental image in which the virtual implant object is placed refers to a multi-dimensional image such as a 2D image or a 3D image which shows teeth arrangement of a patient and is generated to establish an implant surgery plan. Various types of images such as an x-ray image, a CT image, a magnetic resonance imaging (MRI) image, a panoramic image, an oral scan image, an image generated through reconstruction, and an image obtained by matching a plurality of images may be utilized in the implant surgery plan.

The controller 14 according to one embodiment automatically performs maxillary sinus lifting simulation during the implant placement simulation. For example, when the maxillary sinus lifting is necessary due to maxillary sinus involvement by an implant, the controller 14 performs the maxillary sinus lifting simulation and automatically displays maxillary sinus lifting simulation information in a dental image. The maxillary sinus lifting simulation information includes information on an area where maxillary sinus involvement occurs based on the implant and information on an amount of bone graft. The information on the amount of bone graft may be expressed as a volume value of an implant involvement area.

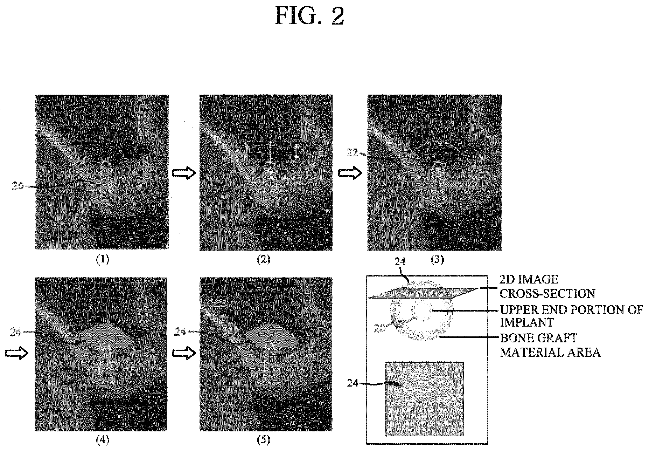

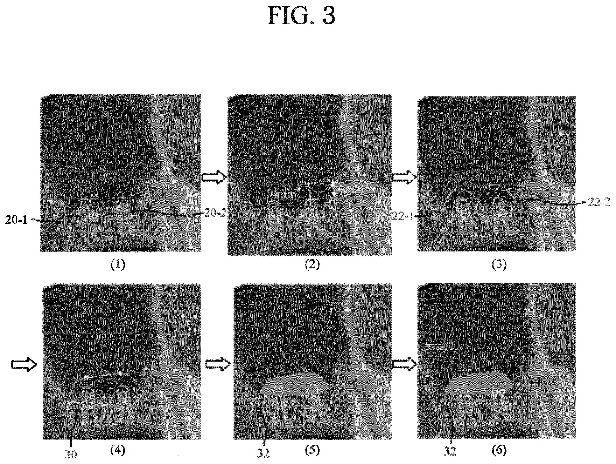

In order to determine whether the maxillary sinus lifting is necessary, the controller 14 sets each of a maxillary sinus simulation area and the implant involvement area. The maxillary sinus simulation area refers to a semispherical area having, as a radius, a length from a reference point of the implant as a start point to a point at a predetermined distance from an upper end of the implant. The implant involvement area refers to an area having a density value less than a predetermined density value in the maxillary sinus simulation area. In response to the implant involving the implant involvement area, the controller 14 may determine that the maxillary sinus lifting is necessary. The setting of the maxillary sinus simulation area and the implant involvement area will be described below with reference to FIGS. 2 and 3.

In response to a change in an implant placement situation, the controller 14 may change the maxillary sinus lifting simulation information according to the changed implant placement situation and automatically display the changed maxillary sinus lifting simulation information in the dental image. For example, in response to a change in implant information, the controller 14 changes the information on the area where the maxillary sinus involvement occurs based on the implant and the information on the amount of bone graft to correspond to the changed implant information and automatically displays the changed information on the area where the maxillary sinus involvement occurs based on the implant and information on the amount of bone graft in the dental image. The implant information may include a length, a diameter, a slope, and the like of the implant. In response to movement of the implant, the controller 14 may move the maxillary sinus simulation area together, and in response to a change in the length of the implant, the controller 14 may change the radius of the maxillary sinus simulation area. According to a change in the maxillary sinus simulation area, the controller 14 may also change the implant involvement area and the information on the amount of bone graft and display the changed implant involvement area and information on the amount of bone graft on the screen.

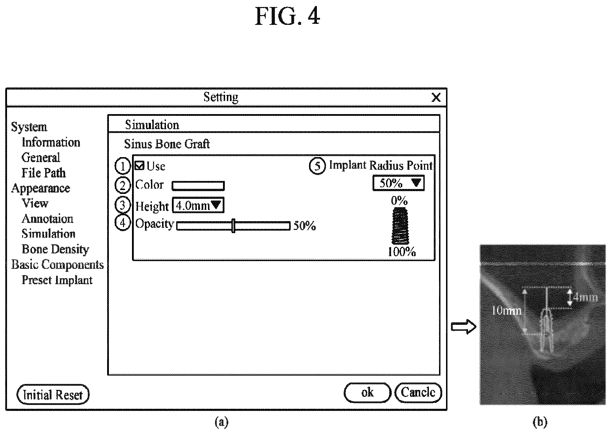

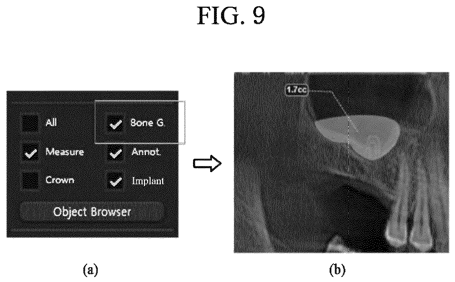

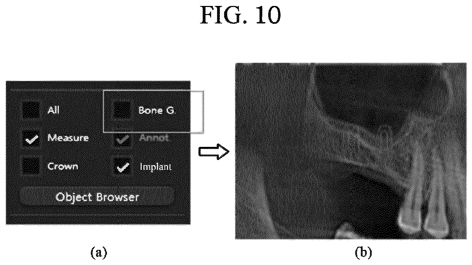

The input device 16 receives a manipulation signal from a user. For example, the input device 16 receives a manipulation signal for user setting on a maxillary sinus lifting simulation setting screen. The maxillary sinus lifting simulation setting screen includes an interface for setting whether to use maxillary sinus lifting simulation, an interface for setting a display color of the implant involvement area, an interface for setting a length from the upper end of the implant to the maxillary sinus simulation area, an interface for setting opacity of the implant involvement area, an interface for setting the reference point of the implant, and the like. The maxillary sinus lifting simulation setting screen will be described below with reference to FIG. 4. The input device 16 may receive a manipulation signal for user setting relating to area showing and area hiding. The setting of the area showing and area hiding will be described below with reference to FIGS. 9 and 10.

The output device 18 displays a screen. Here, the output device 18 may display a dental image for placing a virtual implant object, an implant placement simulation screen on which the implant is placed in the dental image, a maxillary sinus lifting simulation screen, and the like.

The output device 18 may display the implant involvement area to be visually identifiable through the maxillary sinus lifting simulation screen and may also display information on the volume of the implant involvement area. Here, in response to a change in the implant information, the output device 18 may change the implant involvement area and the volume thereof to correspond to the changed implant information and display the changed implant involvement area and volume thereof.

FIG. 2 is a view illustrating image screens according to a maxillary sinus lifting simulation process according to one embodiment of the present disclosure.

Referring to FIG. 2, in (1), software places an implant 20 in a dental image of a patient. A CT image may be used to set an implant placement position. The CT image includes a 3D image, a 2D image, and a panoramic image. The 2D image includes an axial image, a sagittal image or a cross image, a coronal image, and the like. **In** an implant placement operation, the implant may be automatically placed using a reference axis of a crown, adjacent teeth, and bone density information. Using setting information for each tooth number stored in an implant library, the implant 20 of a set size, that is, a set length and a diameter, may be placed at a position corresponding to a selected tooth number.

Then, in (2), the software calculates a length (e.g., 9 mm) from a reference point of the placed implant 20 as a start point to a point at a predetermined distance (e.g., 4mm) from an upper end of the implant 20 and sets the calculated length as a radius. For example, as illustrated in FIG. 2, in a case in which the length of the implant 20 is 10 mm and the predetermined distance is 4 mm, the length 9 mm, which is obtained by adding the value of the predetermined distance 4 mm to 5 mm which is a value of a length from a central point of the implant 20 to the upper end of the implant 20, is set as the radius. Although the central point is set as the reference point in FIG. 2, the reference point may be changed by user setting. The predetermined distance from the upper end of the implant 20 may also be changed by user setting.

Then, in (3), the software sets a maxillary sinus simulation area 22 in a semispherical shape having the calculated length (e.g., 9 mm) as the radius. The maxillary sinus simulation area 22 is generated in a semicircular shape in a 2D cross-section image and is generated in the semispherical shape in a 3D image.

Then, in (4), the software sets an area having a density value less than a predetermined density value in the set maxillary sinus simulation area 22 as an implant involvement area 24 and then, in a case in which the implant 20 involves the implant involvement area 24, displays the implant involvement area 24 on the screen. Here, the implant involvement area 24 is provided in the form of identifiable visual information on the screen to allow the user to check the implant involvement area 24. The identifiable visual information may be displayed using various methods, e.g., displayed with a different color, distinguished by a pattern, or indicated with a separate mark, an outline, or a leader line. The Hounsfield Unit (HU) value may be used for the density value. Here, the implant involvement area 24 may be displayed to have a predetermined opacity value.

Then, in (5), the software calculates and displays the volume of the implant involvement area 24. The volume of the implant involvement area 24 may be provided as a numerical value. The unit of the volume of the implant involvement area 24 may be (with 1cc = 1cm³) or mm³ but is not limited thereto. In FIG. 2, "1.5 cc" is displayed on the screen. The volume information may be displayed in a 2D image, a 3D image, or on a separate screen. Using the volume information of the implant involvement area 24, the user may recognize the amount of bone graft material necessary for the corresponding area. The bone graft material is a material developed to, in the case of bone loss due to disease or trauma, fill the bone in an area where the bone is missing. When, through surgery, the bone graft material is placed into an area where bone is missing, new bone grows around the bone graft material. In dentistry, the bone graft material is used as a material for filling an area where bone is missing when placing an implant.

FIG. 3 is a view illustrating image screens according to a maxillary sinus lifting simulation process according to another embodiment of the present disclosure.

While the case described above with reference to FIG. 2 is a case in which a single implant is placed, FIG. 3 corresponds to a case in which two or more implants are consecutively placed.

In (1), the software places implants in a dental image of a patient. Two or more implants, e.g., a first implant 20-1 and a second implant 20-2 as illustrated in FIG. 3, are consecutively placed.

In (2), when placing the two consecutive implants 20-1 and 20-2, the software calculates a length from a reference point of each of the implants 20-1 and 20-2 as a start point to a point at a predetermined distance from an upper end of each of the implants 20-1 and 20-2 and sets the calculated length as a radius. For example, in a case in which the length of the second implant 20-2 is 12 mm and the predetermined distance is 4 mm as illustrated in FIG. 3, the length 10 mm, which is obtained by adding the value of the predetermined distance 4 mm to 6 mm which is a value of a length from a central point of the second implant 20-2 to the upper end of the second implant 20-2, is set as the radius. Although the central point is set as the reference point in FIG. 3, the reference point may be changed by user setting. The predetermined distance from the upper end of each of the implants 20-1 and 20-2 may also be changed by user setting.

Then, in (3), the software sets maxillary sinus simulation areas 22-1 and 22-2 in semispherical shapes having the respective calculated lengths as the radius. The two maxillary sinus simulation areas 22-1 and 22-2 are generated in a semicircular shape in a 2D cross-section image and are generated in the semispherical shape in a 3D image. Here, the two maxillary sinus simulation areas 22-1 and 22-2 may at least partially overlap with each other.

**In** (4), in response to the two maxillary sinus simulation areas 22-1 and 22-2 at least partially overlapping with each other, the software connects end points of the two maxillary sinus simulation areas 22-1 and 22-2 to each other to generate a single maxillary sinus simulation area 30. For example, the software detects a first end point, where a vertical line segment starting from the central point of the first implant 20-1 meets a first maxillary sinus simulation area 22-1, and a second end point, where a vertical line segment starting from the central point of the second implant 20-2 meets a second maxillary sinus simulation area 22-2, and connects the first end point and the second end point. Here, the single maxillary sinus simulation area 30 which has a semicylindrical shape and a height equal to a height of a line segment generated by connecting the first end point and the second end point is newly generated.

Then, in (5), the software sets an area having a density value less than a predetermined density value in the set single maxillary sinus simulation area 30 as an implant involvement area 32 and then, in a case in which the implants 20-1 and 20-2 involve the implant involvement area 32, displays the implant involvement area 32 on the screen. Here, the implant involvement area 32 is provided in the form of identifiable visual information on the screen to allow the user to check the implant involvement area 32 from images. The identifiable visual information may be displayed using various methods, e.g., displayed with a different color, distinguished by a pattern, or indicated with a separate mark, an outline, or a leader line. The HU value may be used for the density value. Here, the implant involvement area 32 may be displayed to have a predetermined opacity value.

Then, in (6), the software calculates and displays the volume of the implant involvement area 32. The volume of the implant involvement area 32 may be provided as a numerical value. The unit of the volume of the implant involvement area 32 may be cc or mm³ but is not limited thereto. In FIG. 3, "2.1 cc" is displayed on the screen. The volume information may be displayed in a 2D image, a 3D image or on a separate screen. Using the volume information of the implant involvement area 32, the user may recognize the amount of bone graft necessary for the maxillary sinus lifting. Here, the volume information is information in which multiple pieces of information are combined into one.

FIG. 4 is a view illustrating a setting screen for maxillary sinus lifting simulation and a use example thereof according to one embodiment of the present disclosure.

In more detail, FIG. 4A illustrates a maxillary sinus lifting simulation setting screen, and FIG. 4B illustrates a use example of maxillary sinus lifting simulation setting.

The maxillary sinus lifting simulation setting screen includes: 1) an interface for setting whether to use maxillary sinus lifting simulation; 2) an interface for setting a display color of the implant involvement area; 3) an interface for setting a length from the upper end of the implant to the maxillary sinus simulation area; 4) an interface for setting opacity of the implant involvement area; and 5) an interface for setting the reference point of the implant. FIG. 4B illustrates an example in which the length set through the interface for setting the length from the upper end of the implant to the maxillary sinus simulation area ("3)") is 4 mm, and a reference point set through the interface for setting the reference point of the implant ("5)") is the central point of the implant that corresponds to 50%.

Through the maxillary sinus lifting simulation setting, the user can arbitrarily set whether to use maxillary sinus lifting simulation, the display color of the implant involvement area, the height from the end of the implant, the opacity of the expressed color, and a start point of the radius based on the implant. Through the setting, the user can deal with various parameters for each case and situation.

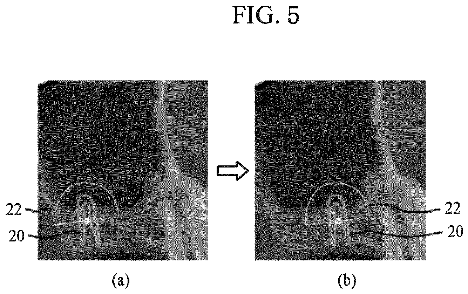

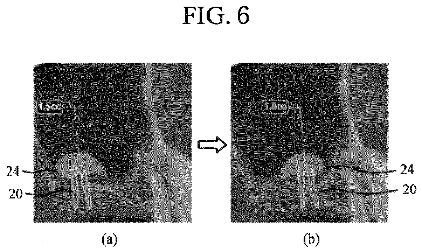

FIG. 5 is a view illustrating image screens showing movement of a maxillary sinus simulation area according to movement of an implant according to one embodiment of the present disclosure, and FIG. 6 is a view illustrating image screens displaying movement of an implant involvement area and a change in an amount of bone graft according to movement of an implant.

Referring to FIG. 5, as illustrated in FIG. 5A, the software generates the maxillary sinus simulation area 22 based on the implant 20. Then, as illustrated in FIG. 5B, in a case in which the implant 20 moves, the software also identically moves the maxillary sinus simulation area 22 according to the corresponding implant 20. The movement of the implant includes a change in the slope of the implant.

In response to the maxillary sinus simulation area 22 moving according to the movement of the implant 20 as illustrated in FIG. 5, the software also changes the position of the implant involvement area 24 and displays the changed position and also changes the volume value of the implant involvement area 24 and displays the changed volume value as illustrated in FIG. 6. For example, it can be seen from FIG. 6 that the volume value is changed from 1.5 cc in FIG. 6A to 1.6 cc in FIG. 6B.

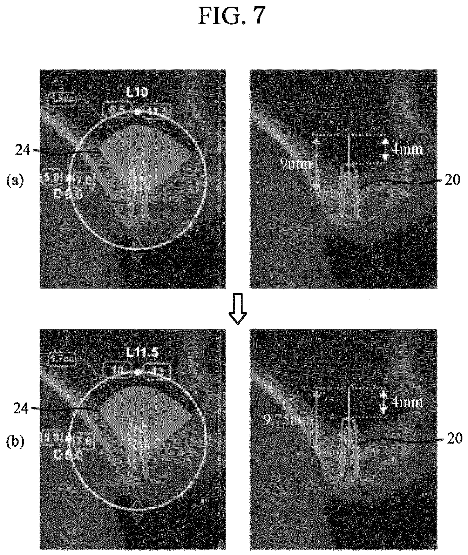

FIG. 7 is a view illustrating image screens of the implant involvement area according to a change in the length of the implant according to one embodiment of the present disclosure.

In more detail, FIG. 7A illustrates an image screen before a change in the length of the implant, and FIG. 7B illustrates an image screen after the change in the length of the implant.

Referring to FIG. 7A, in a case in which, based on the center line of the implant 20, a length from the central point of the implant 20 to a point at a predetermined distance (e.g., 4mm) from the upper end of the implant 20 is designated as a length of a set area, before a change in the length of the implant, a maxillary sinus simulation area generated when an implant having a length of 10 mm is placed has a semispherical shape whose radius has a length of 9 mm. Therefore, a simulation is performed based on the semispherical shape whose radius is 9 mm, and the implant involvement area 24 and the volume value thereof (1.5 cc) are displayed based on the semispherical shape.

Referring to FIG. 7B, in a case in which the length of the implant is changed to 11.5 mm, the maxillary sinus simulation area has a semispherical shape whose radius has a length of 9.75 mm. Since the length of the radius of the maxillary sinus simulation area is increased by 0.75 mm as compared to when the length of the implant is 10 mm, the area of the displayed implant involvement area 24 is also larger, and the displayed volume thereof is also larger (is increased from 1.5 cc to 1.7 cc).

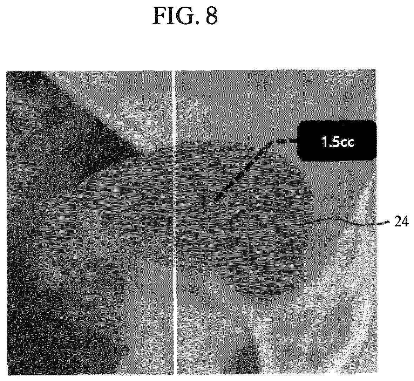

FIG. 8 is a view illustrating a screen automatically displaying maxillary sinus lifting simulation information in a 3D image according to one embodiment of the present disclosure.

Referring to FIG. 8, in a case in which, during the implant placement simulation, maxillary sinus involvement by an implant occurs, the software three-dimensionally displays maxillary sinus lifting simulation information, which includes the implant involvement area 24, on a 3D image screen. As in a 2D cross-section image, the implant involvement area 24 is provided in the form of identifiable visual information on the screen to allow the user to check the implant involvement area 24. The identifiable visual information may be displayed using various methods, e.g., displayed with a different color, distinguished by a pattern, or indicated with a separate mark, an outline, or a leader line. Here, the software may also display the volume value (e.g., 1.5 cc) of the implant involvement area 24.

FIG. 9 is a view illustrating image screens in a case in which screen showing is selected by a user according to one embodiment of the present disclosure.

Referring to FIG. 9, the user may set an implant involvement area to be shown on the screen by selecting area showing. For example, in a case in which the user has selected area showing on the setting screen as illustrated in FIG. 9A, the implant involvement area and information on a predicated amount of bone graft may be displayed together on an image screen of FIG. 9B.

FIG. 10 is a view illustrating image screens in a case in which screen hiding is selected by the user according to one embodiment of the present disclosure.

Referring to FIG. 10, the user may set an implant involvement area to be hidden on the screen by selecting area hiding. For example, in a case in which the user has selected area hiding on the setting screen as illustrated in FIG. 10A, the implant involvement area and information on a predicated amount of bone graft are not displayed on an image screen of FIG. 10B.

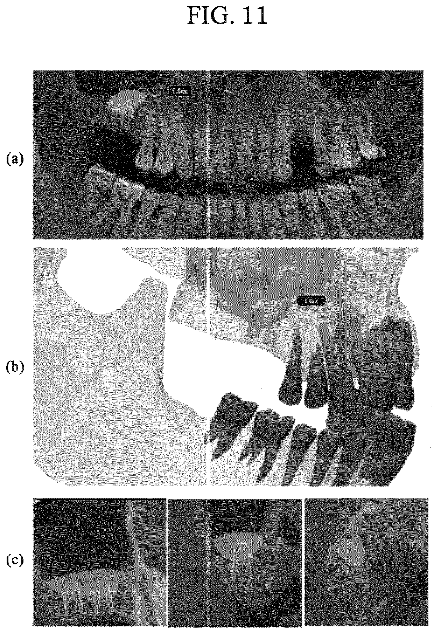

FIG. 11 is a view illustrating image screens for maxillary sinus lifting simulation according to one embodiment of the present disclosure.

Referring to FIG. 11, the software may display maxillary sinus lifting simulation information on all screens including: a panoramic image screen shown in FIG. 11A; a 3D image screen shown in FIG. 11B; and 2D cross-section image screens (axial, coronal, sagittal screens) shown in FIG. 11C. Here, the displayed maxillary sinus lifting simulation information includes information on an area where maxillary sinus involvement occurs based on the implant and information on a volume value thereof.

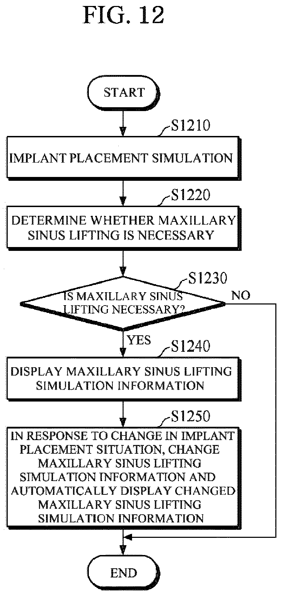

FIG. 12 is a view illustrating a flowchart of a maxillary sinus lifting simulation method according to one embodiment of the present disclosure.

Referring to FIG. 12, the software performs implant placement simulation in a dental image of a patient (S1210).

Then, during the implant placement simulation, the software determines whether maxillary sinus lifting is necessary due to maxillary sinus involvement by an implant (S1220). In the determining of whether the maxillary sinus lifting is necessary (S1220), the software may set a maxillary sinus simulation area based on the implant placed in the dental image. For example, the software sets the maxillary sinus simulation area in a semispherical shape having, as a radius, a length from a reference point of the implant as a start point to a point at a predetermined distance from an upper end of the implant. In a case in which two implants are consecutively placed, in response to two maxillary sinus simulation areas at least partially overlapping with each other, the software may connect end points of the two maxillary sinus simulation areas to each other to set a single maxillary sinus simulation area. Then, the software sets an implant involvement area within the set maxillary sinus simulation area. Here, the software may calculate a density value of the maxillary sinus simulation area and set an area having a density value less than a predetermined density value in the maxillary sinus simulation area as the implant involvement area. Then, the software determines whether the implant involves the implant involvement area and, in response to the implant involving the implant involvement area, determines that the maxillary sinus lifting is necessary.

**In** response to the maxillary sinus lifting being determined as necessary (yes in S1230), the software performs maxillary sinus lifting simulation and automatically displays maxillary sinus lifting simulation information in the dental image (S1240). The maxillary sinus lifting simulation information may include information on an area where maxillary sinus involvement occurs based on the implant and information on an amount of bone graft. The information on the amount of bone graft may be expressed as a volume value of the implant involvement area.

Then, in response to a change in an implant placement situation, the software may change the maxillary sinus lifting simulation information according to the changed implant placement situation and automatically display the changed maxillary sinus lifting simulation information in the dental image (S1250). For example, in response to a change in implant information, the software changes the information on the area where the maxillary sinus involvement occurs based on the implant and the information on the amount of bone graft to correspond to the changed implant information and automatically displays the changed information on the area where the maxillary sinus involvement occurs based on the implant and information on the amount of bone graft in the dental image. In response to movement of the implant, the software may move the maxillary sinus simulation area together, and, in response to a change in a length of the implant, the software may change the radius of the maxillary sinus simulation area. The software may also change the implant involvement area and the information on the amount of bone graft according to the change in the maxillary sinus simulation area.

The present disclosure has been described above on the basis of embodiments thereof. Those of ordinary skill in the art to which the present disclosure pertains should understand that the present disclosure can be implemented in modified forms within the scope not departing from essential characteristics of the present disclosure. Therefore, the embodiments disclosed herein should be considered as illustrative, instead of limiting. The invention is defined by the appended claims.

## Claims

1. A maxillary sinus lifting simulation method performed by a maxillary sinus lifting simulation apparatus (1), the maxillary sinus lifting simulation method comprising:
an operation of performing (S1210) implant placement simulation in a dental image of a patient;
an operation of, during the implant placement simulation, setting (S1220) a maxillary sinus simulation area (22) based on a distance from a reference point position of the implant (20) in a semispherical shape having, as a radius, the length from the reference point position of the implant (20) as a start point to a point at a predetermined distance from an upper end of the implant (20);
an operation of setting an implant involvement area (24) based on a density value within the set maxillary sinus simulation area (22), including: an operation of calculating a density value of the set maxillary sinus simulation area (22), wherein the density value refers to a Hounsfield Unit value; and an operation of setting an area having a density value less than a predetermined density value in the maxillary sinus simulation area (22) as the implant involvement area (24); and
an operation of determining (S1230) whether maxillary sinus lifting is necessary based on whether the implant involvement area (24) overlaps with the implant (20), wherein the implant involvement area (24) is an area with a density value less than a predetermined density value in the maxillary sinus simulation area (22); and
an operation of automatically performing (S1240) maxillary sinus lifting simulation only in a case in which the maxillary sinus lifting is necessary.

2. The maxillary sinus lifting simulation method of claim 1, further comprising:
an operation of automatically displaying maxillary sinus lifting simulation information, which includes information on an area where maxillary sinus involvement occurs based on the implant (20) and information on an amount of bone graft, in the dental image; and
an operation of, in response to a change in an implant placement situation, changing (S1250) the maxillary sinus lifting simulation information according to the changed implant placement situation and automatically displaying the changed maxillary sinus lifting simulation information in the dental image.

3. The maxillary sinus lifting simulation method of claim **1,** wherein:
in the operation of the setting of the maxillary sinus simulation area (22), in a case in which two implants (20-1, 20-2) are consecutively placed, in response to two maxillary sinus simulation areas (22-1, 22-2) at least partially overlapping with each other, end points of the two maxillary sinus simulation areas (22-1, 22-2) are connected to each other to set a single maxillary sinus simulation area (30); and
in the setting of the implant involvement area (32), a single implant involvement area (32) that the two implant (20-1, 20-2) involve is set within the set single maxillary sinus simulation area (30).

4. The maxillary sinus lifting simulation method of claim 2, wherein the operation of the automatically displaying of the maxillary sinus lifting simulation information in the dental image includes:
an operation of displaying the implant involvement area (24) in the dental image; and
an operation of calculating and displaying a volume of the implant involvement area (24).

5. The maxillary sinus lifting simulation method of claim 2, wherein, in the operation of the changing of the maxillary sinus lifting simulation information and the automatically displaying of the changed maxillary sinus lifting simulation information in the dental image, in response to a change in implant information, the information on the area (24) where the maxillary sinus involvement occurs based on the implant (20) and the information on the amount of bone graft are changed to correspond to the changed implant information and are automatically displayed in the dental image.

6. The maxillary sinus lifting simulation method of claim 2, wherein, in the operation of the changing of the maxillary sinus lifting simulation information and the automatically displaying of the changed maxillary sinus lifting simulation information in the dental image, in response to movement of the implant (20), the maxillary sinus simulation area (22) is moved together, in response to a change in a length of the implant (20), the radius of the maxillary sinus simulation area (22) is changed, and then the information on the area (24) where the maxillary sinus involvement occurs based on the implant (20) and the information on the amount of bone graft are changed to correspond to the movement of the maxillary sinus simulation area (22) or the change in the radius and are automatically displayed in the dental image.

7. The maxillary sinus lifting simulation method of claim 1, further comprising an operation of providing a maxillary sinus lifting simulation setting screen for setting at least one of an interface for setting whether to use maxillary sinus lifting simulation, an interface for setting a display color of an implant involvement area, an interface for setting a length from an upper end of the implant (20) to a maxillary sinus simulation area (22), an interface for setting opacity of the implant involvement area (22), and an interface for setting a reference point of the implant (22).

8. The maxillary sinus lifting simulation method of claim 1, further comprising:
an operation of receiving a manipulation signal for user setting relating to area showing and area hiding; and
an operation of, in a case in which an area showing setting is selected according to the user setting, displaying the implant involvement area (24) and information on a predicated amount of bone graft in the dental image and, in a case in which an area hiding setting is selected, not displaying the implant involvement area (24) and the information on the predicated amount of bone graft in the dental image.

9. A maxillary sinus lifting simulation apparatus (1) comprising:
- a data acquisition device (10) configured to acquire a dental image of a patient;
- a controller (14) configured to:
- perform implant placement simulation in the dental image of the patient,
- during the implant placement simulation, set a maxillary sinus simulation area (22) in a semispherical shape having, as a radius, a length from a reference point position of the implant (20) as a start point to a point at a predetermined distance from an upper end of the implant (20),
- set an implant involvement area (24) based on a density value within the set maxillary sinus simulation area (22), including calculating a density value of the set maxillary sinus simulation area (22), wherein the density value refers to a Hounsfield Unit value, and
- determine whether maxillary sinus lifting is necessary based on whether the implant involvement area (24) overlaps with the implant (20), wherein the implant involvement area (24) is an area with a density value less than a predetermined density value in the maxillary sinus simulation area (24), and
- automatically perform maxillary sinus lifting simulation only in a case in which the maxillary sinus lifting is necessary; and
- an output device (18) configured to automatically display the maxillary sinus lifting simulation information in the dental image.

10. The maxillary sinus lifting simulation apparatus (1) of claim 9, wherein the controller (14) is configured to automatically output maxillary sinus lifting simulation information, which includes information on an area (24) where maxillary sinus involvement occurs based on the implant (20) and information on an amount of bone graft, in the dental image, and, in response to a change in an implant placement situation, to change the maxillary sinus lifting simulation information according to the changed implant placement situation and automatically output the changed maxillary sinus lifting simulation information in the dental image.

11. The maxillary sinus lifting simulation apparatus (1) of claim 9, wherein: in a case in which two implants (20-1, 20-2) are consecutively placed, in response to two maxillary sinus simulation areas (22-1, 22-2) at least partially overlapping with each other, the controller (14) is configured to connect end points of the two maxillary sinus simulation areas (22-1, 22-2) to each other to set a single maxillary sinus simulation area (30) and to set a single implant involvement area (32) that the two implant (22-1, 22-2) involve within the set single maxillary sinus simulation area (30).

12. The maxillary sinus lifting simulation apparatus (1) of claim 9, wherein the output device (18) is configured to display the implant involvement area (24) and a volume thereof in the dental image and, in response to a change in implant information, to change the implant involvement area (24) and the volume thereof to correspond to the changed implant information and to display the changed implant involvement area (24) and volume thereof in the dental image.

13. The maxillary sinus lifting simulation apparatus (1) of claim 9, wherein the output device (18) is configured to provide a maxillary sinus lifting simulation setting screen for setting at least one of an interface for setting whether to use maxillary sinus lifting simulation, an interface for setting a display color of the implant involvement area (24), an interface for setting a length from the upper end of the implant (20) to the maxillary sinus simulation area (22), an interface for setting opacity of the implant involvement area (24), and an interface for setting the reference point of the implant (20).

## Patentansprüche

1. Kieferhöhlenanhebungssimulationsverfahren, durchgeführt von Kieferhöhlenanhebungs-Simulationsvorrichtung (1), aufweisend:
eine Operation des Durchführens (S1210) einer Implantatplatzierungssimulation in einer Zahnaufnahme eines Patienten;
eine Operation des Einstellens (S1220), während der Implantatplatzierungssimulation, eines Kieferhöhlensimulationsbereichs (22) basierend auf einem Abstand von Referenzpunktposition des Implantats (20) in halbkugelförmiger Gestalt mit als Radius der Länge von der Referenzpunktposition des Implantats (20) als Startpunkt zu einem Punkt in vorbestimmtem Abstand von Oberende des Implantats (20);
eine Operation des Einstellens eines Implantatbeteiligungsbereichs (24) basierend auf einem Dichtewert innerhalb des eingestellten Kieferhöhlensimulationsbereichs (22), mit: einer eine Operation des Berechnens eines Dichtewerts des eingestellten Kieferhöhlensimulationsbereichs (22), wobei der Dichtewert sich auf einen Hounsfield-Einheiten-Wert (HU) bezieht; und einer Operation des Einstellens eines Bereichs mit einem Dichtewert kleiner als ein vorbestimmter Dichtewert im Kieferhöhlensimulationsbereich (22) als Implantatbeteiligungsbereich (24); und
eine Operation des Bestimmens (S1230), ob eine Kieferhöhlenanhebung erforderlich ist, basierend darauf, ob der Implantatbeteiligungsbereich (24) sich mit dem Implantat (20) überlappt, wobei der Implantatbeteiligungsbereich (24) ein Bereich mit einem Dichtewert kleiner als ein vorbestimmter Dichtewert im Kieferhöhlensimulationsbereich (22) ist; und
eine Operation des automatischen Durchführens (S1240) einer Kieferhöhlenanhebungssimulation nur für den Fall, dass die Kieferhöhlenanhebung erforderlich ist.

2. Kieferhöhlenanhebungssimulationsverfahren nach Anspruch 1, ferner aufweisend:
eine Operation des automatischen Anzeigens von Kieferhöhlenanhebungssimulationsinformationen, die Informationen über einen Bereich enthalten, in dem Kieferhöhlenbeteiligung in Bezug auf das Implantat (20) auftritt, und Informationen über eine Menge von Knochentransplantat, in der Zahnaufnahme; und
eine Operation des Änderns (S1250), in Reaktion auf eine Änderung einer Implantatplatzierungssituation, der Kieferhöhlenanhebungssimulationsinformationen gemäß der geänderten Implantatplatzierungssituation und automatischen Anzeigens der geänderten Kieferhöhlenanhebungssimulationsinformationen in der Zahnaufnahme.

3. Kieferhöhlenanhebungssimulationsverfahren nach Anspruch 1, wobei:
in der Operation des Einstellens des Kieferhöhlensimulationsbereichs (22), für den Fall, dass zwei Implantate (20-1, 20-2) aufeinanderfolgend angeordnet sind, in Reaktion auf zwei Kieferhöhlensimulationsbereiche (22-1, 22-2), die zumindest teilweise miteinander überlappen, Endpunkte der zwei Kieferhöhlensimulationsbereiche (22-1, 22-2) miteinander verbunden werden, um einen einzelnen Kieferhöhlensimulationsbereich (30) einzustellen; und
bei dem Einstellen des Implantatbeteiligungsbereichs (32) ein einzelner Implantatbeteiligungsbereich (32), den die zwei Implantate (20-1, 20-2) betreffen, innerhalb des eingestellten einzelnen Kieferhöhlensimulationsbereichs (30) eingestellt wird.

4. Kieferhöhlenanhebungssimulationsverfahren nach Anspruch 2, wobei die Operation des automatischen Anzeigens der Kieferhöhlenanhebungssimulationsinformationen in der Zahnaufnahme Folgendes aufweist:
einen Operation des Anzeigens des Implantatbeteiligungsbereichs (24) in der Zahnaufnahme; und
eine Operation des Berechnens und Anzeigens eines Volumens des Implantat-Beteiligungsbereichs (24).

5. Kieferhöhlenanhebungssimulationsverfahren nach Anspruch 2, wobei in der Operation des Änderns der Kieferhöhlenanhebungssimulationsinformationen und des automatischen Anzeigens der geänderten Kieferhöhlenanhebungssimulationsinformationen in der Zahnaufnahme in Reaktion auf eine Änderung von Implantatsinformationen die Informationen über den Bereich (24), in dem die Kieferhöhlenbeteiligung in Bezug auf das Implantat (20) auftritt, und die Informationen über die Menge von Knochentransplantat so geändert werden, dass sie den geänderten Implantatsinformationen entsprechen, und automatisch in der Zahnaufnahme angezeigt werden.

6. Kieferhöhlenanhebungssimulationsverfahren nach Anspruch 2, wobei in dem Vorgang des Änderns der Kieferhöhlenanhebungssimulationsinformationen und des automatischen Anzeigens der geänderten Kieferhöhlenanhebungssimulationsinformationen in der Zahnaufnahme in Reaktion auf ein Bewegen des Implantats (20) der Kieferhöhlensimulationsbereich (22) mitbewegt wird, in Reaktion auf eine Änderung einer Länge des Implantats (20) der Radius des Kieferhöhlensimulationsbereichs (22) geändert wird und danach die Informationen über den Bereich (24), in dem die Kieferhöhlenbeteiligung in Bezug auf das Implantat (20) auftritt, und die Informationen über die Menge von Knochentransplantat so geändert werden, dass sie dem Bewegen des Kieferhöhlensimulationsbereichs (22) oder der Änderung des Radius entsprechen, und automatisch in der Zahnaufnahme angezeigt werden.

7. Kieferhöhlenanhebungssimulationsverfahren nach Anspruch 1, ferner aufweisend eine Operation des Bereitstellens eines Kieferhöhlenanhebungssimulationseinstellbildschirms zum Einstellen von zumindest einer aus: eine Schnittstelle zum Einstellen, ob eine Kieferhöhlenanhebungssimulation verwendet wird, eine Schnittstelle zum Einstellen einer Anzeigefarbe eines Implantatbeteiligungsbereichs, eine Schnittstelle zum Einstellen einer Länge von einem Oberende des Implantats (20) zu einem Kieferhöhlensimulationsbereich (22), eine Schnittstelle zum Einstellen einer Deckkraft des Implantatbeteiligungsbereichs (22) und eine Schnittstelle zum Einstellen eines Referenzpunkts des Implantats (22).

8. Kieferhöhlenanhebungssimulationsverfahren nach Anspruch 1, ferner aufweisend:
eine Operation des Empfangens eines Betätigungssignals für Benutzereinstellung bezüglich Bereichsanzeige und Bereichsausblendung; und
eine Operation des Anzeigens, für den Fall, dass gemäß der Benutzereinstellung eine Bereichsanzeigeneinstellung ausgewählt ist, des Implantatbeteiligungsbereichs (24) und von Informationen über eine vorhergesagte Menge von Knochentransplantat in der Zahnaufnahme und, für den Fall, dass eine Bereichsausblendungseinstellung ausgewählt ist, Nicht-Anzeigens des Implantatbeteiligungsbereichs (24) und der Informationen über die vorhergesagte Menge von Knochentransplantat in der Zahnaufnahme.

9. Kieferhöhlenanhebungssimulationsvorrichtung (1) aufweisend:
- eine Datenerfassungsvorrichtung (10), die eingerichtet ist, eine Zahnaufnahme eines Patienten zu erfassen;
- ein Steuergerät (14), das eingerichtet ist:
- eine Implantatplatzierungssimulation in der Zahnaufnahme des Patienten durchzuführen,
- während der Implantatplatzierungssimulation einen Kieferhöhlensimulationsbereich (22) in halbkugelförmiger Gestalt mit als Radius einer Länge von Referenzpunktposition des Implantats (20) als Startpunkt zu einem Punkt in vorbestimmtem Abstand von Oberende des Implantats (20) einzustellen,
- einen Implantatbeteiligungsbereich (24) basierend auf Dichtewert innerhalb des eingestellten Kieferhöhlensimulationsbereichs (22) einzustellen, einschließlich Berechnens eines Dichtewerts des eingestellten Kieferhöhlensimulationsbereichs (22), wobei der Dichtewert sich auf Hounsfield-Einheiten-Wert (HU) bezieht, und
- zu bestimmen, ob eine Kieferhöhlenanhebung erforderlich ist, basierend darauf, ob der Implantatbeteiligungsbereich (24) sich mit dem Implantat (20) überlappt, wobei der Implantat-Beteiligungsbereich (24) ein Bereich mit einem Dichtewert kleiner als ein vorbestimmter Dichtewert im KieferhöhlenSimulationsbereich (24) ist, und
- automatisches eine Kieferhöhlenanhebungssimulation nur für den Fall durchzuführen, dass die Kieferhöhlenanhebung erforderlich ist; und
ein Ausgabegerät (18), das eingerichtet ist, die Kieferhöhlenanhebungssimulationsinformationen automatisch in der Zahnaufnahme anzuzeigen.

10. Kieferhöhlenanhebungssimulationsvorrichtung (1) nach Anspruch 9, wobei das Steuergerät (14) dazu eingerichtet ist, Kieferhöhlenanhebungssimulationsinformationen, die Informationen über einen Bereich (24) enthalten, in dem Kieferhöhlenbeteiligung in Bezug auf das Implantat (20) auftritt, und Informationen über eine Menge von Knochentransplantat, automatisch in der Zahnaufnahme auszugeben und, in Reaktion auf eine Änderung einer Implantatplatzierungssituation, die Kieferhöhlenanhebungssimulationsinformationen gemäß der geänderten Implantatplatzierungssituation zu ändern und die geänderten Kieferhöhlenanhebungssimulationsinformationen automatisch in der Zahnaufnahme auszugeben.

11. Kieferhöhlenanhebungssimulationsvorrichtung (1) nach Anspruch 9, wobei, für den Fall, dass zwei Implantate (22-1, 22-2) aufeinanderfolgend angeordnet sind, in Reaktion auf zwei Kieferhöhlensimulationsbereiche (22-1, 22-2), die zumindest teilweise miteinander überlappen, das Steuergerät (14) eingerichtet ist, Endpunkte der zwei Kieferhöhlensimulationsbereiche (22-1, 22-2) miteinander zu verbinden, um einen einzelnen Kieferhöhlensimulationsbereich (30) einzustellen, und innerhalb des eingestellten einzelnen Kieferhöhlensimulationsbereichs (30) einen einzelnen Implantatbeteiligungsbereich (32), den die zwei Implantate (22-1, 22-2) betreffen, einzustellen.

12. Kieferhöhlenanhebungssimulationsvorrichtung (1) nach Anspruch 9, wobei das Ausgabegerät (18) eingerichtet ist, den Implantatbeteiligungsbereich (24) und dessen Volumen in der Zahnaufnahme anzuzeigen und in Reaktion auf eine Änderung von Implantatsinformationen den Implantatbeteiligungsbereich (24) und dessen Volumen so zu ändern, dass sie den geänderten Implantatsinformationen entsprechen, und den geänderten Implantatbeteiligungsbereich (24) und dessen Volumen in der Zahnaufnahme anzuzeigen.

13. Kieferhöhlenanhebungssimulationsvorrichtung (1) nach Anspruch 9, wobei das Ausgabegerät (18) dazu eingerichtet ist, einen Kieferhöhlenanhebungssimulationseinstellbildschirm bereitzustellen zum Einstellen von zumindest einer aus: einer Schnittstelle zum Einstellen, ob Kieferhöhlenanhebungssimulation verwendet wird, einer Schnittstelle zum Einstellen einer Anzeigefarbe des Implantatbeteiligungsbereichs (24), eine Schnittstelle zum Einstellen einer Länge von einem Oberende des Implantats (20) zu dem Kieferhöhlensimulationsbereich (22), eine Schnittstelle zum Einstellen einer Deckkraft des Implantatbeteiligungsbereichs (24) und eine Schnittstelle zum Einstellen des Referenzpunkts des Implantats (20).

## Revendications

1. Procédé de simulation de surélévation de sinus maxillaire réalisé par un appareil de simulation de surélévation de sinus maxillaire (1), le procédé de de simulation de surélévation de sinus maxillaire comprenant :
une opération pour réaliser (S1210) une simulation de placement d'implant dans une image dentaire d'un patient ;
durant la simulation de placement d'implant, une opération pour régler (S1220) une zone de simulation de sinus maxillaire (22) sur la base d'une distance partant d'une position de pointe de référence de l'implant (20) dans une forme semi-sphérique, comme rayon, la longueur allant de la position de point de référence de l'implant (20) comme point de départ jusqu'à un point à une distance prédéterminée à partir d'une extrémité supérieure de l'implant (20) ;
une opération pour régler une zone d'implication d'implant (24) sur la base d'une valeur de densité dans les limites de la zone de simulation de sinus maxillaire réglée (22), incluant : une opération pour calculer une valeur de densité de la zone de simulation de sinus maxillaire réglée (22), dans lequel la valeur de densité se réfère à une valeur d'unité Hounsfield, et une opération pour régler une zone présentant une valeur de densité inférieure à une valeur de densité prédéterminée dans la zone de simulation de sinus maxillaire (22) comme zone d'implication d'implant (24), et
une opération pour déterminer (2130) si oui ou non la surélévation de sinus maxillaire est nécessaire sur la base de si oui ou non la zone d'implication d'implant (24) présente un chevauchement avec l'implant, dans lequel la zone d'implication de l'implant (24) est une zone présentant une valeur de densité inférieure à une valeur de densité prédéterminée dans la zone de simulation de sinus maxillaire (22), et
une opération pour réaliser automatiquement (S1240) une simulation de surélévation de sinus maxillaire uniquement dans un cas où la surélévation de sinus maxillaire est nécessaire.

2. Procédé de simulation de surélévation de sinus maxillaire selon la revendication 1, comprenant en outre :
une opération pour afficher automatiquement des informations de simulation de surélévation de sinus maxillaire, lesquelles incluent des informations sur une zone où l'implication de sinus maxillaire se produit sur la base de l'implant (20) et des informations sur une quantité de greffe osseuse, dans l'image dentaire, et
en réaction à une modification de la situation de placement d'implant, une opération pour modifier (S1250) les informations de simulation de surélévation de sinus maxillaire conformément à la situation de placement d'implant modifiée, et pour afficher automatiquement les informations de simulation de surélévation de sinus maxillaire modifiées dans l'image dentaire.

3. Procédé de simulation de surélévation de sinus maxillaire selon la revendication 1, dans lequel :
lors de l'opération pour régler la zone de simulation de sinus maxillaire (22), dans un cas où deux implants (20-1, 20-2) sont placés de manière consécutive, en réaction à deux zones de simulation de sinus maxillaire (22-1, 22-2) se chevauchant entre elles au moins partiellement, des points d'extrémité des deux zones de simulation de sinus maxillaire (22-1, 22-2) sont reliés entre eux pour régler une zone de simulation de surélévation de sinus maxillaire unique (30), et
lors du réglage de la zone d'implication d'implant (32), une zone d'implication d'implant unique (32), laquelle implique les deux implants (20-1, 20-2), est réglée dans les limites de la zone de simulation de sinus maxillaire unique réglée (30).

4. Procédé de simulation de surélévation de sinus maxillaire selon la revendication 2, dans lequel l'opération pour afficher automatiquement des informations de simulation de surélévation de sinus maxillaire dans l'image dentaire inclut :
une opération pour afficher la zone d'implication d'implant (24) dans l'image dentaire, et
une opération pour calculer et afficher un volume de la zone d'implication d'implant (24).

5. Procédé de simulation de surélévation de sinus maxillaire selon la revendication 2, dans lequel lors de l'opération pour modifier les informations de simulation de surélévation de sinus maxillaire et pour afficher automatiquement les informations de simulation de surélévation de sinus maxillaire modifiées dans l'image dentaire, en réaction à une modification des informations d'implant, les informations sur la zone (24) où l'implication de sinus maxillaire se produit sur la base de l'implant (20) et les informations sur la quantité de greffe osseuse sont modifiées pour correspondre aux informations d'implant modifiées, et sont automatiquement affichées dans l'image dentaire.

6. Procédé de simulation de surélévation de sinus maxillaire selon la revendication 2, dans lequel lors de l'opération pour modifier les informations de simulation de surélévation de sinus maxillaire et pour afficher automatiquement les informations de simulation de surélévation de sinus maxillaire modifiées dans l'image dentaire, en réaction au déplacement de l'implant (20), la zone de simulation de sinus maxillaire (22) est déplacée conjointement, en réaction à une modification d'une longueur de l'implant (20) ; le rayon de la zone de simulation de sinus maxillaire (22) est modifié, puis les informations sur la zone (24) où l'implication de sinus maxillaire se produit sur la base de l'implant (20) et les informations sur la quantité de greffe osseuse sont modifiées pour correspondre au déplacement de la zone de simulation de sinus maxillaire (22) ou à la modification du rayon, et sont automatiquement affichées dans l'image dentaire.

7. Procédé de simulation de surélévation de sinus maxillaire selon la revendication 1, comprenant en outre une opération pour fournir un écran de réglage de simulation de surélévation de sinus maxillaire pour régler au moins un élément parmi une interface pour régler si oui ou non il faut utiliser la simulation de surélévation de sinus maxillaire, une interface pour régler une couleur d'affichage d'une zone d'implication d'implant, une interface pour régler une longueur allant d'une extrémité supérieure de l'implant (20) à une zone de simulation de sinus maxillaire (22), une interface pour régler une opacité de la zone d'implication d'implant (22), et une interface pour régler un point de référence de l'implant (22).

8. Procédé de simulation de surélévation de sinus maxillaire selon la revendication 1, comprenant en outre :
une opération pour recevoir un signal de manipulation pour un réglage utilisateur concernant l'affichage de zone et le masquage de zone, et
dans un cas où un réglage d'affichage de zone est sélectionné conformément à un réglage utilisateur, une opération pour afficher la zone d'implication d'implant (24) et des informations sur une quantité prédite de greffe osseuse dans l'image dentaire, et dans un cas où un réglage de masquage de zone est sélectionné, pour ne pas afficher la zone d'implication d'implant (24) et les informations sur la quantité prédite de greffe osseuse dans l'image dentaire.

9. Appareil de simulation de surélévation de sinus maxillaire (1), comprenant :
- un dispositif d'acquisition de données (10) configuré pour acquérir une image dentaire d'un patient ;
- un contrôleur (14) configuré pour :
- réaliser une simulation de placement d'implant dans l'image dentaire du patient ;
- durant la simulation de placement d'implant, régler une zone de simulation de sinus maxillaire (22) dans une forme semi-sphérique présentant, comme rayon, la longueur allant d'une position de point de référence de l'implant (20) comme point de départ jusqu'à un point à une distance prédéterminée à partir d'une extrémité supérieure de l'implant (20) ;
- régler une zone d'implication d'implant (24) sur la base d'une valeur de densité dans les limites de la zone de simulation de sinus maxillaire (22), incluant le calcul d'une valeur de densité de la zone de simulation de sinus maxillaire réglée (22), dans lequel la valeur de densité se réfère à une valeur d'unité Hounsfield, et
- déterminer si oui ou non la surélévation de sinus maxillaire est nécessaire sur la base de si oui ou non la zone d'implication d'implant (24) présente un chevauchement avec l'implant (20), dans lequel la zone d'implication de l'implant (24) est une zone présentant une valeur de densité inférieure à une valeur de densité prédéterminée dans la zone de simulation de sinus maxillaire (24), et
réaliser automatiquement une simulation de surélévation de sinus maxillaire uniquement dans un cas où la surélévation de sinus maxillaire est nécessaire, et
- un dispositif de sortie (18) configuré pour afficher automatiquement les informations de simulation de surélévation de sinus maxillaire dans l'mage dentaire.

10. Appareil de simulation de surélévation de sinus maxillaire (1) selon la revendication 9, dans lequel le contrôleur (14) est configuré pour produire automatiquement des informations de simulation de surélévation de sinus maxillaire, lesquelles incluent des informations sur une zone (24) où l'implication de sinus maxillaire se produit sur la base de l'implant (20) et des informations sur une quantité de greffe osseuse, dans l'image dentaire, et en réaction à une modification de la situation de placement d'implant, pour modifier les informations de simulation de surélévation de sinus maxillaire conformément à la situation de placement d'implant modifiée, et pour produire automatiquement les informations de simulation de surélévation de sinus maxillaire modifiées dans l'image dentaire.

11. Appareil de simulation de surélévation de sinus maxillaire (1) selon la revendication 9, dans lequel dans un cas où deux implants (20-1, 20-2) sont placés de manière consécutive, en réaction à deux zones de simulation de sinus maxillaire (22-1, 22-2) se chevauchant entre elles au moins partiellement, le contrôleur (14) est configuré pour relier des points d'extrémité des deux zones de simulation de sinus maxillaire (22-1, 22-2) entre eux pour régler une zone de simulation de surélévation de sinus maxillaire unique (30), et pour régler une zone d'implication d'implant(32), laquelle implique les deux implants (20-1, 20-2) dans les limites de la zone de simulation de sinus maxillaire unique réglée (30).

12. Appareil de simulation de surélévation de sinus maxillaire (1) selon la revendication 9, dans lequel le dispositif de sortie (18) est configuré pour afficher la zone d'implication d'implant (24) et un volume de celle-ci dans l'image dentaire, et en réaction à une modification des informations d'implant, pour modifier la zone d'implication d'implant (24) et le volume de celle-ci pour une correspondance avec les informations d'implant modifiées, et pour afficher la zone d'implication d'implant modifiée (24) et le volume de celle-ci dans l'image dentaire.

13. Appareil de simulation de surélévation de sinus maxillaire (1) selon la revendication 9, dans lequel le dispositif de sortie (18) est configuré pour fournir un écran de réglage de simulation de surélévation de sinus maxillaire pour régler au moins un élément parmi une interface pour régler si oui ou non il faut utiliser la simulation de surélévation de sinus maxillaire, une interface pour régler une couleur d'affichage d'une zone d'implication d'implant (24), une interface pour régler une longueur allant d'une extrémité supérieure de l'implant (20) à une zone de simulation de sinus maxillaire (22), une interface pour régler une opacité de la zone d'implication d'implant (24), et une interface pour régler le point de référence de l'implant (20).
